# EUROPEAN PATENT APPLICATION

(11) **EP 4 417 687 A1**
(43) Date of publication of application: **21.08.2024**
(21) Application number: 21960423.8
(22) Date of filing: 22.11.2021
(51) Int. Cl.: C12N 5/09, C12Q 1/02

(54) **CULTURE MEDIUM FOR PRIMARY OVARIAN CANCER CELLS, CULTURE METHOD AND APPLICATION THEREOF**

(30) Priority: 15.10.2021 CN 202111201798
(71) Applicant: Precedo Pharmaceuticals Co., Ltd., Hefei, Anhui 230094 (CN)
(72) Inventor: LIU, Qingsong, Hefei, Anhui 230031 (CN); HE, Yuying, Hefei, Anhui 230031 (CN); HUANG, Tao, Hefei, Anhui 230031 (CN); CHEN, Cheng, Hefei, Anhui 230031 (CN)
(74) Representative: Abel & Imray LLP
(86) International application number: PCT/CN2021/131967
(87) International publication number: WO 2023/060696

(57) **Abstract**

A culture medium for primary ovarian cancer cells, an *in vitro* culture method and an application thereof. The culture medium contains: an MST1/2 kinase inhibitor; a Rho kinase inhibitor, which is selected from at least one among Y27632, Fasudil and H-1152; an insulin-transferrin-selenium supplement; insulin; prostaglandin E2; an epidermal growth factor; gastrin; an insulin-like growth factor-1; cholera toxin; amphiregulin; N2; and B27. Compared with existing culture methods, the *in vitro* culture using said culture medium has higher amplification efficiency. Using the culture medium for the culturing of primary ovarian cancer cells may maintain the morphological structure and pathological features of primary tissues, and improve the success rate and survival rate of the cultured primary ovarian cancer cells.

## Description

### Technical Field

The invention relates to the field of biomedical technology, and in particular to a culture medium and application thereof, and more specifically to a culture medium for primary ovarian cancer cells, and a culture method and application thereof.

### Background of the Invention

Ovarian cancer refers to a malignant tumor disease that occurs in the ovary, which is one of the common malignant tumors of female reproductive organs, and has an incidence second only to cervical cancer and uterine body cancer. Epithelial ovarian cancer is the most common type of ovarian cancer, followed by malignant ovarian germ cell tumor. Epithelial ovarian cancer has the highest mortality rate among all types of gynecological tumors and may pose a serious threat to women's lives. Ovarian cancer is often asymptomatic in the early stage, but gastrointestinal symptoms such as lower abdominal discomfort, bloating, and decreased appetite may occur in the late stage. The main treatment options for ovarian cancer include surgical resection, drug therapy, and radiotherapy, and the overall prognosis is poor.

Chemotherapy is one of the main treatments for ovarian tumors. Although there are many chemotherapy drugs currently available for clinical use, the clinical treatment effectiveness of ovarian tumors is only about 25%. The main reason is that most chemotherapy drug regimens used by patients are based on the experience of clinicians, undergoing trial-evaluation-medication change and trial-reevaluation without considering the individual differences of patients, not only fails to improve the therapeutic effect of the drugs, but also misses the best treatment period, leading to the tumor entering the advanced stage. In addition, patients are burdened with drug side effects and extremely high medical costs throughout the treatment process.

Therefore, establishing a primary tumor model *in vitro* and using it to conduct efficient drug screening experiments is a promising solution. At present, the main method for establishing *in vitro* culture models of primary ovarian tumors is the Patient-Derived tumor Xenograft (PDX) model, which involves transplanting tumor cells from patients into nude mice and examining the therapeutic effects of different anti-tumor drugs. However, the PDX method also has some shortcomings, such as, species differences between humans and mice; long test cycle (more than 4 weeks); high cost (more than 200,000); false positives and false negatives, etc.

### Summary of the Invention

In order to solve the aforementioned technical problems, the invention provides a culture medium for primary ovarian cancer cells, and a culture method *in vitro* and application thereof.

One aspect of the invention is to provide a culture medium for primary ovarian cancer cells, comprising a MST1/2 kinase inhibitor, at least one Rho kinase inhibitor selected from the group consisting of Y27632, fasudil, and H-1152; insulin-transferrin-selenium supplement; insulin; prostaglandin E2; epidermal growth factor (EGF); gastrin; insulin-like growth factor-1; cholera toxin; amphiregulin; N2; and B27.

Wherein the MST1/2 kinase inhibitor comprises a compound of Formula (I) or a pharmaceutically acceptable salt, or a solvate thereof, 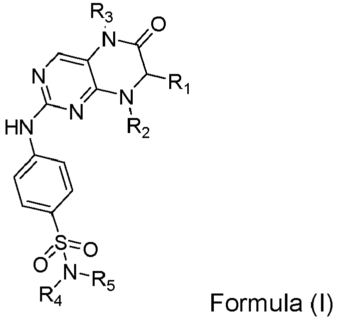 wherein,
R₁ is selected from C1-C6 alkyl, C3-C6 cycloalkyl, C4-C8 cycloalkylalkyl, C2-C6 spirocycloalkyl, and aryl (e.g., phenyl and naphthyl, etc.) optionally substituted with 1-2 independent R₆, aryl C1-C6 alkyl (e.g., phenylmethyl, etc.) optionally substituted with 1-2 independent R₆ and heteroaryl (e.g., thienyl, etc.) optionally substituted with 1-2 independent R₆;
R₂ and R₃ are each independently selected from C1-C6 alkyl, preferably C1-C3 alkyl, more preferably methyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl (the heterocyclyl is selected from e.g., piperidyl, tetrahydropyranyl, etc.);
R₆ is selected from halogen (preferably fluoro and chloro, more preferably fluoro), C1-C6 alkyl (preferably methyl), C1-C6 alkoxy (preferably methoxy), and C1-C6 haloalkyl (preferably trifluoromethyl).

In a preferable embodiment, the MST1/2 kinase inhibitor comprises a compound of Formula (Ia) or a pharmaceutically acceptable salt, or a solvate thereof, 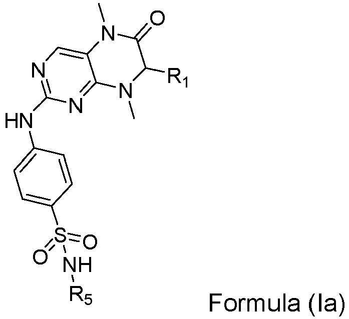 wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆; more preferably, R₁ is phenyl optionally substituted with 1-2 independent R₆;
R₅ is selected from hydrogen, C1-C6 alkyl, and C3-C6 cycloalkyl; more preferably, R₅ is hydrogen;
R₆ is independently selected from halogen, C1-C6 alkyl, and C1-C6 haloalkyl; more preferably, R₆ is fluoro, methyl or trifluoromethyl.

Preferably, the MST1/2 kinase inhibitor is at least one selected from the following compounds or a pharmaceutically acceptable salt, or a solvate thereof.

| | | | |
|---|---|---|---|
| 1 | 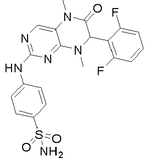 | 2 | 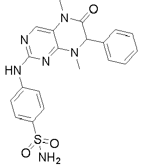 |
| 3 | 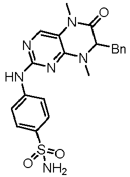 | 4 | 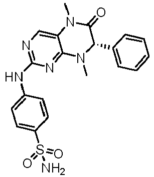 |
| 5 | 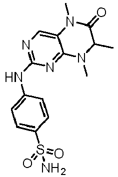 | 6 | 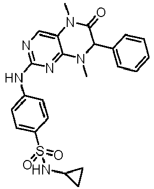 |
| 7 | 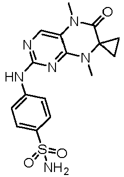 | 8 | 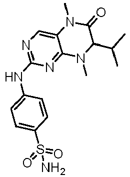 |
| 9 | 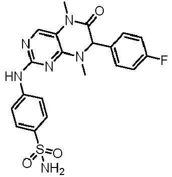 | 10 | 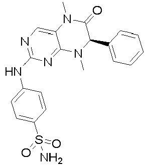 |
| 11 | 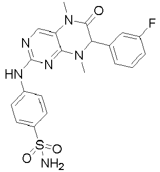 | 12 | 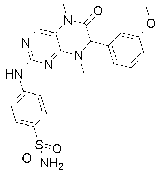 |
| 13 | 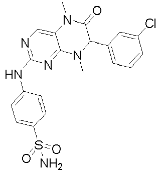 | 14 | 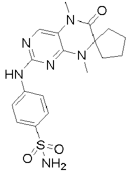 |
| 15 | 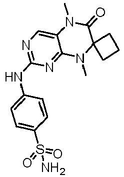 | 16 | 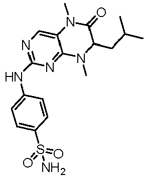 |
| 17 | 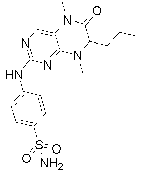 | 18 | 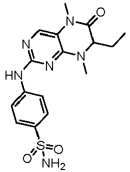 |
| 19 | 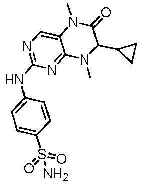 | 20 | 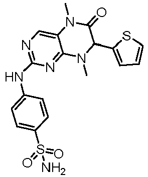 |
| 21 | 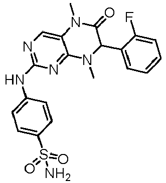 | 22 | 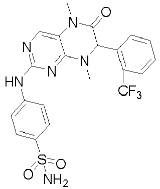 |
| 23 | 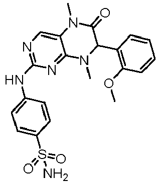 | 24 | 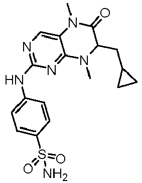 |
| 25 | 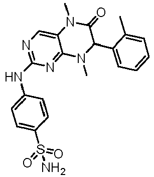 | 26 | 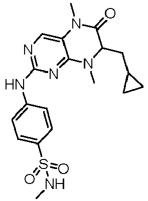 |
| 27 | 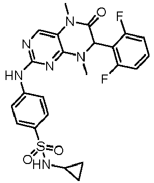 | 28 | 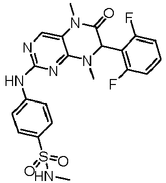 |
| 29 | 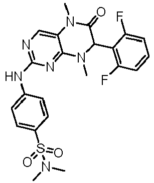 | 30 | 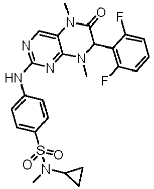 |
| 31 | 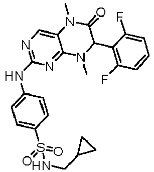 | 32 | 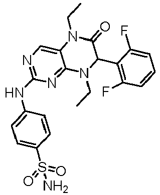 |
| 33 | 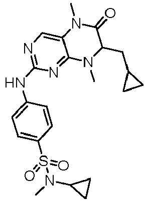 | 34 | 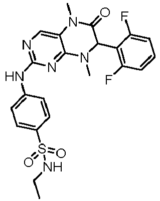 |
| 35 | 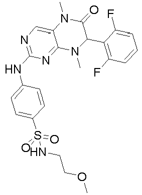 | 36 | 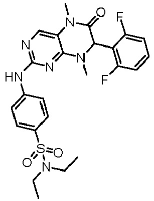 |
| 37 | 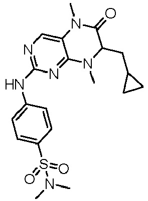 | 38 | 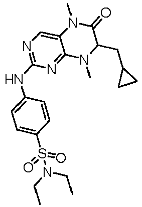 |
| 39 | 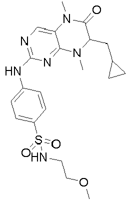 | 40 | 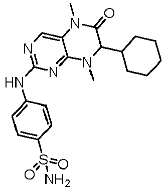 |
| 41 | 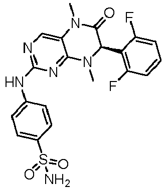 | 42 | 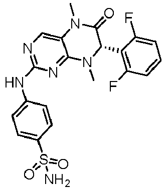 |
| 43 | 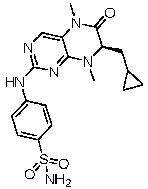 | 44 | 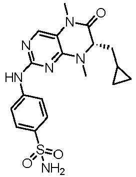 |
| 45 | 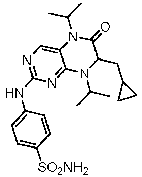 | 46 | 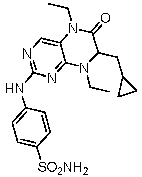 |
| 47 | 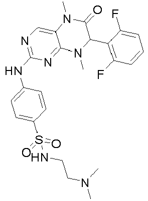 | 48 | 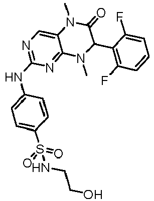 |
| 49 | 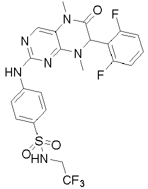 | 50 | 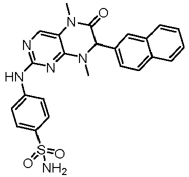 |
| 51 | 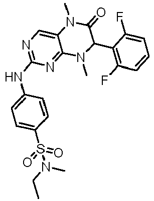 | 52 | 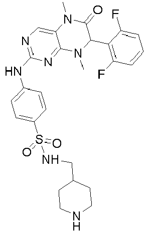 |
| 53 | 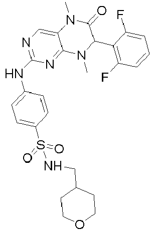 | 54 | 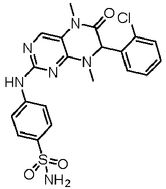 |
| 55 | 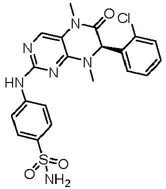 | 56 | 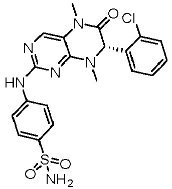 |
| 57 | 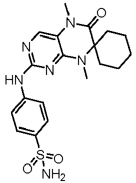 | 58 | 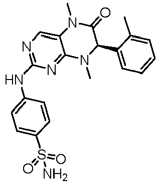 |
| 59 | 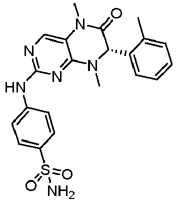 | | |

Most preferably, the MST1/2 kinase inhibitor of the invention is Compound 1.

In an embodiment of the invention, the amount of the components in the culture medium of the invention satisfies any one or more or all of the following conditions:
(1) the amount of the MST1/2 kinase inhibitor in the culture medium is 2.5-20 µM;
(2) the amount of the Rho kinase inhibitor in the culture medium is 2.5-20 µM;
(3) the volume ratio of the insulin-transferrin-selenium supplement relative to the culture medium is 1:800-1:50;
(4) the amount of the insulin in the culture medium is 1-27 µg/mL;
(5) the amount of the prostaglandin E2 in the culture medium is 2.5-10 µM;
(6) the amount of the epidermal growth factor in the culture medium is 2.5-40 ng/mL;
(7) the amount of the gastrin in the culture medium is 1-9 nM;
(8) the amount of the insulin-like growth factor-1 in the culture medium is 25-100 ng/mL;
(9) the amount of the cholera toxin in the culture medium is 0.05-0.8 µg/mL;
(10) the amount of the amphiregulin in the culture medium is 1-81 ng/mL;
(11) the volume ratio of the B27 additive relative to the culture medium is preferably 1:25-1:400;
(12) the volume ratio of the N2 additive relative to the culture medium is preferably 1:50-1:400.

In the embodiment of the invention, the culture medium further comprises an initial medium selected from the group consisting of DMEM/F12, DMEM, F12 or RPMI-1640; and one or more antibiotics selected from the group consisting of streptomycin/penicillin, amphotericin B and Primocin.

In a preferred embodiment, in case of using streptomycin/penicillin as the antibiotics, the concentration range of streptomycin is 25-400 µg/mL, the concentration range of penicillin is 25-400 U/mL; in case of using amphotericin B as the antibiotics, the concentration range is 0.25-4 µg/mL; and in case of using Primocin as the antibiotics, the concentration range is 25-400 µg/mL.

According to the second aspect, the invention also provides a method for culturing primary ovarian cancer cells *in vitro.* In the method for culturing primary ovarian cancer cells *in vitro* of the invention, the primary ovarian cancer cells are cultured *in vitro* using the culture medium for primary ovarian cancer cells of the invention.

The method for culturing primary ovarian cancer cells *in vitro* of the invention comprises the following steps:

### 1. Isolating the primary ovarian cancer cells

(1) ovarian cancer tissue samples are separated and added with a 1:3 ratio of Basal Medium and tissue digestion solution (note: the amount of tissue digestion solution added is about 5-10 mL tissue digestion solution for 1 g of tumor tissue), and placed in a constant-temperature shaker for digestion, at a digestion temperature of 4-37°C and a speed of 200-350 rpm;
(2) digestion may be terminated until it is fully completed, i.e. no obvious tissue debris is found, and the digestion time is 3-6 hours;
(3) the supernatant is discarded after centrifugation with a centrifugation speed of 1200-1600 rpm for 2-6 minutes, and Basal Medium is added to resuspend the cells for later use.

Wherein, the formulation of the Basal Medium comprises an initial medium selected from the group consisting of DMEM/F12, DMEM, F12 or RPMI-1640; and one or more antibiotics selected from the group consisting of streptomycin/penicillin, amphotericin B and Primocin. The formulation of the tissue digestion solution comprises 1640 medium, collagenase II (1-2 mg/mL), collagenase IV (1-2 mg/mL), DNase (50-100 U/mL), hyaluronidase (0.5-1 mg/mL), calcium chloride (1-5 mM), bovine serum albumin BSA (5-10 mg/mL).

### 2. Culturing the cells using the culture medium for primary ovarian cancer cells of the invention

The Basal Medium is mixed with matrigel (Corning^{®}, 356231) in a ratio of 25:1 to 100:1 on ice. After plating the cells, the plates are incubated in a 5% CO₂ incubator at 37°C for 25 to 35 minutes, and the supernatant is discarded. The primary ovarian cancer cells obtained in the above step 1 are resuspended with the culture medium for primary ovarian cancer cells of the invention, and the cells are counted. The cell density is adjusted to 2-8×10⁴ cells /cm². The culture medium for primary ovarian cancer cells of the invention is added, and the cells are cultured in an incubator.

In yet another aspect, the invention also provides a drug screening method for ovarian cancer disease, which includes the following steps:
(1) culturing primary ovarian cancer cells using the method for culturing primary ovarian cancer cells of the invention;
(2) selecting the drug to be tested and diluting it into desired concentration gradients;
(3) adding the drug which has been diluted into various concentration gradients to the cells cultured in step(1);
(4) detecting the cell viability.

The technical solution of the invention can achieve the following technical effects:
(1) the success rate of culturing primary ovarian cancer cells is improved, and the invention allows culturing tumor tissues from various sample sources such as epithelial cancer, malignant germ cell tumor, stromal tumor and metastatic tumor, and the success rate is 80% or more;
(2) the primary ovarian cancer cells cultured *in vitro* is ensured to maintain the pathological characteristics of patients;
(3) the cultured primary ovarian cancer cells are not interfered by stromal cells such as fibroblasts and adipocytes;
(4) the expanding efficiency is high, and primary ovarian cancer cells can be successfully cultured in about a week, and the expanded primary ovarian cancer cells have the ability of continuous passage;
(5) the cost for culture is controllable, as the culture medium does not require expensive factors such as Wnt agonists;
(6) the culture medium does not contain serum, and can avoid co-culture of primary cells with stromal cells, and also solves a series of problems existing in traditional culture protocols such as serum-containing and co-culture with stromal cells;
(7) the primary ovarian cancer cells cultured by the above technology are large in number and highly homogenized, making them suitable for high-throughput screening of new candidate compounds and providing high-throughput drug sensitivity *in vitro* for patients.

### Description of Drawings

Figure 1 is a graph showing the effects of different combinations of added factors in the culture medium for primary ovarian cancer cells on the growth of primary ovarian cancer cells.
Figures 2A-2L are graphs showing the effects of different concentrations of each added factor in the culture medium for primary ovarian cancer cells on the growth of primary ovarian cancer cells.
Figures 3A-3F are photographs taken under a microscope of primary ovarian cancer cells cultured using the culture medium for primary ovarian cancer cells of the invention.
Figures 4A-4G show the immunohistochemical results of original ovarian cancer tissue cells.
Figures 5A-5G are immunohistochemical results of primary ovarian cancer cells obtained by culturing original ovarian cancer tissue cells to the fourth passage using the culture medium for primary ovarian cancer cells of the invention.
Figures 6A-6C show the cell growth curves of primary ovarian cancer cells cultured using the culture medium for primary ovarian cancer cells of the invention, the prior art culture medium and the commercial culture medium respectively.
Figures 7A-7F show the results of drug screening of primary ovarian cancer cells cultured to different passages using the culture medium for primary ovarian cancer cells of the invention.

### Detailed Description of the Invention

In order to better understand the invention, it is further described below in combination with the embodiments and the drawings. The following examples are provided only for the purpose of illustrating, but not for limiting the invention.

### [Preparation Examples of MST1/2 Kinase Inhibitors]

In the specification, MST1/2 kinase inhibitor refers to any inhibitor that directly or indirectly negatively regulates MST1/2 signaling. Generally, MST1/2 kinase inhibitors reduce the activity of MST1/2 kinase by, for example, binding to the same. Since MST1 and MST2 have similar structures, MST1/2 kinase inhibitors may be, for example, compounds that bind to MST1 or MST2 and reduce the activity thereof.

### 1. Preparation of MST1/2 kinase inhibitor Compound 1

### 4-((7-(2,6-Difluorophenyl)-5,8-dimethyl-6-oxo-5,6,7,8-tetrahydropteridin-2-yl) amino)benzsulfamide 1

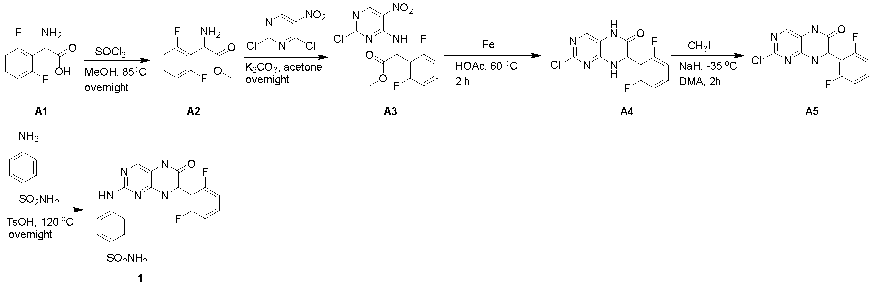

Methyl 2-amino-2-(2,6-difluorophenyl)acetate (A2): 2-amino-2-(2,6-difluorophenyl)acetic acid (2.0 g) and then methanol (30 mL) were added into a round bottom flask, followed by addition of thionyl chloride (1.2 mL) dropwise under an ice bath. The reaction system was reacted overnight at 85°C. After the completion of the reaction, the system was evaporated under reduced pressure to dry the solvent, and the obtained white solid was directly used in the next step.

Methyl 2-((2-chloro-5-nitropyrimidin-4-yl)amino)-2-(2,6-difluorophenyl) acetate (A3): methyl 2-amino-2-(2,6-difluorophenyl)acetate (2 g) and then acetone (30 mL) and potassium carbonate (2.2 g) were added into a round bottom flask, and then the system was cooled to -10°C with an ice salt bath, and then a solution of 2,4-dichloro-5-nitropyrimidine (3.1 g) in acetone was slowly added. The reaction system was stirred overnight at room temperature. After the completion of the reaction, the reaction mixture was filtered, the solvent was removed from the filtrate under reduced pressure, and the residue was purified by pressurized silica gel column chromatography to obtain compound A3. LC/MS: M+H 359.0.

2-Chloro-7-(2,6-difluorophenyl)-7,8-dihydropteridin-6(5H)-one (A4): methyl 2-((2-chloro-5-nitropyrimidin-4-yl)amino)-2-(2,6-difluorophenyl)acetate (2.5 g) and then acetic acid (50 mL) and iron powder (3.9 g) were added into a round bottom flask. The reaction system was stirred at 60°C for two hours. After the completion of the reaction, the reaction system was evaporated under reduced pressure to dry the solvent, and the resultant was neutralized to alkaline with saturated sodium bicarbonate solution and was extracted with ethyl acetate. The organic phase was washed with water and saturated brine and dried with anhydrous sodium sulfate. The organic phase was filtered and evaporated to dryness under reduced pressure to obtain a crude product. The crude product was washed with diethyl ether to obtain compound A4. LC/MS: M+H 297.0.

2-Chloro-7-(2,6-difluorophenyl)-5,8-dimethyl-7,8-dihydropteridin-6(5H)-one (A5): 2-chloro-7-(2,6-difluorophenyl)-7,8-dihydropteridin-6(5H)-one (2 g) and N,N-dimethylacetamide (10 mL) were added into a round bottom flask, and cooled to -35°C, followed by addition of iodomethane (0.9 mL) and then sodium hydride (615 mg), and the reaction system was stirred for two hours. After the completion of the reaction, the reaction mixture was quenched with water, and extracted with ethyl acetate. The organic phase was washed with water and saturated brine, respectively, and dried with anhydrous sodium sulfate. The organic phase was filtered and evaporated to dryness under reduced pressure to obtain a crude product. The crude product was washed with diethyl ether to obtain compound A5. LC/MS: M+H 325.0.

4-((7-(2,6-Difluorophenyl)-5,8-dimethyl-6-oxo-5,6,7,8-tetrahydropteridin-2-yl )amino)benzsulfamide (1): 2-chloro-7-(2,6-difluorophenyl)-5,8-dimethyl-7,8-dihydropteridin-6(5H)-one (100 mg), sulfanilamide (53 mg), p-toluenesulfonic acid (53 mg) and sec-butanol (5 mL) were added into a round bottom flask. The reaction system was stirred at 120°C overnight. After the completion of the reaction, the reaction mixture was filtered, and washed with methanol and diethyl ether to obtain compound 1. LC/MS: M+H 461.1.

### 2. Preparation of other MST1/2 inhibitor compounds of the invention

Other MST1/2 inhibitor compounds of the invention were synthesized via the method similar to that of Compound 1, and their structures and mass spectrum data are shown in the following table.

| No. | Compound | MS(ESI) m/z(M+1)+ | No. | Compound | MS(ESI) m/z(M+1)+ |
|---|---|---|---|---|---|
| 1 | 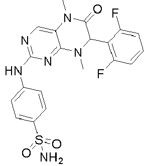 | 461.1 | 2 | 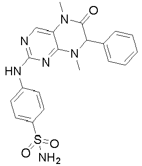 | 425.14 |
| 3 | 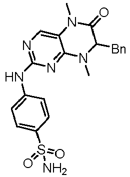 | 439.15 | 4 | 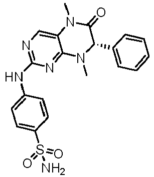 | 425.14 |
| 5 | 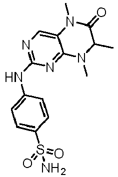 | 363.12 | 6 | 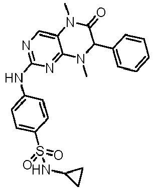 | 465.17 |
| 7 | 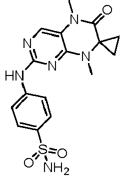 | 375.12 | 8 | 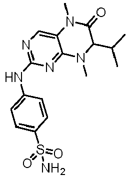 | 391.16 |
| 9 | 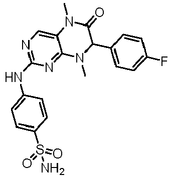 | 443.13 | 10 | 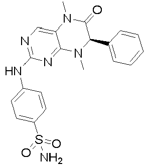 | 425.14 |
| 11 | 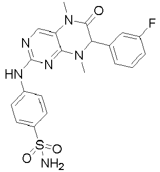 | 443.13 | 12 | 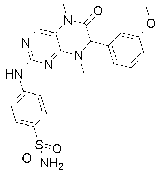 | 455.15 |
| 13 | 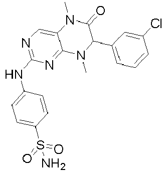 | 459.10 | 14 | 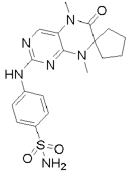 | 403.15 |
| 15 | 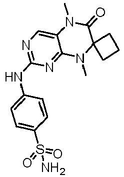 | 389.14 | 16 | 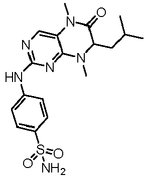 | 405.17 |
| 17 | 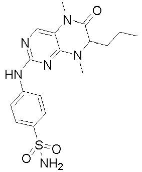 | 391.15 | 18 | 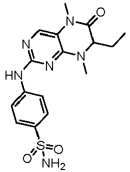 | 377.14 |
| 19 | 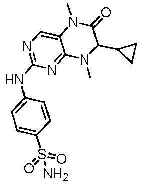 | 389.14 | 20 | 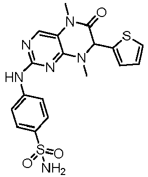 | 431.09 |
| 21 | 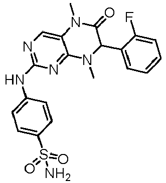 | 443.13 | 22 | 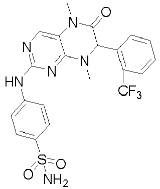 | 493.12 |
| 23 | 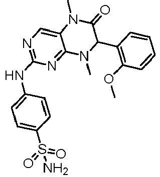 | 455.15 | 24 | 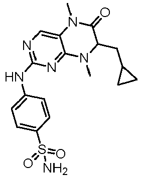 | 403.15 |
| 25 | 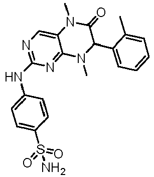 | 439.15 | 26 | 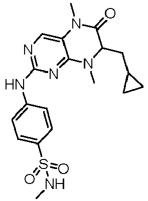 | 417.17 |
| 27 | 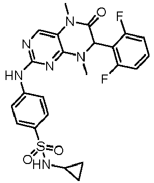 | 501.15 | 28 | 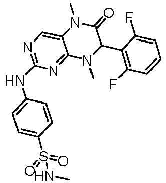 | 475.13 |
| 29 | 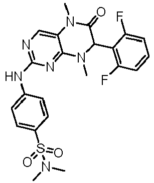 | 489.15 | 30 | 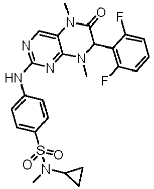 | 515.16 |
| 31 | 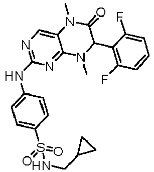 | 515.16 | 32 | 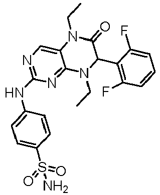 | 489.15 |
| 33 | 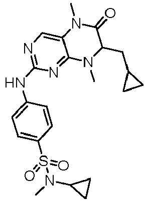 | 457.20 | 34 | 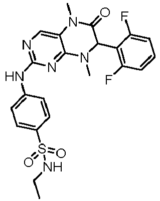 | 489.15 |
| 35 | 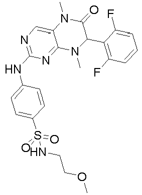 | 519.16 | 36 | 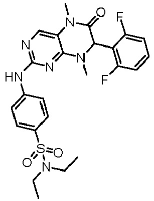 | 517.18 |
| 37 | 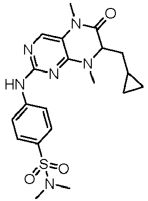 | 431.18 | 38 | 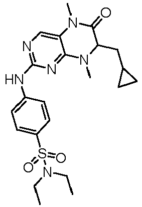 | 459.21 |
| 39 | 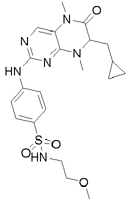 | 461.19 | 40 | 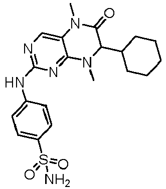 | 431.19 |
| 41 | 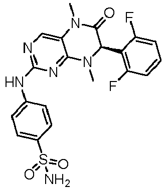 | 461.12 | 42 | 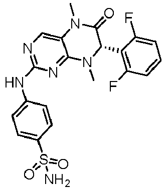 | 461.12 |
| 43 | 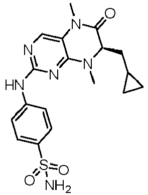 | 403.15 | 44 | 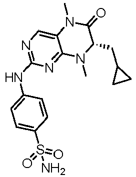 | 403.15 |
| 45 | 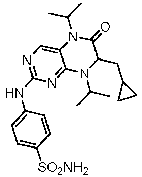 | 459.21 | 46 | 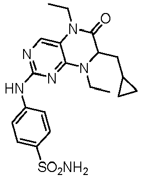 | 431.18 |
| 47 | 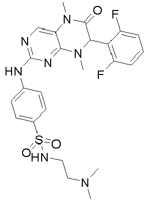 | 532.19 | 48 | 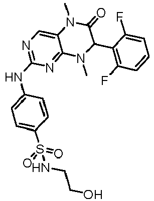 | 505.14 |
| 49 | 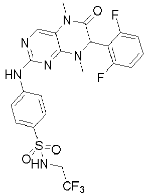 | 543.12 | 50 | 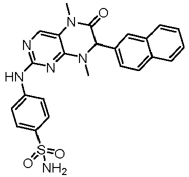 | 475.16 |
| 51 | 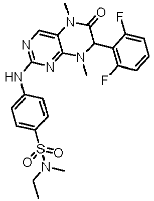 | 503.17 | 52 | 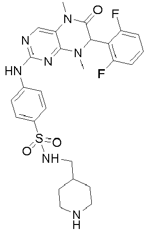 | 558.21 |
| 53 | 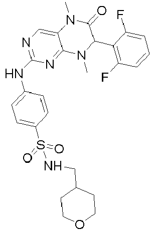 | 559.19 | 54 | 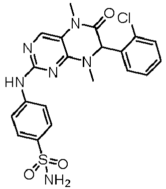 | 459.10 |
| 55 | 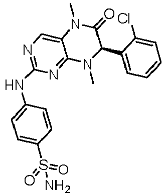 | 459.10 | 56 | 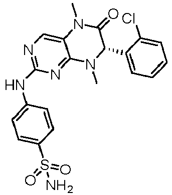 | 459.10 |
| 57 | 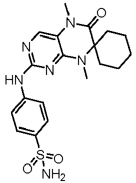 | 417.17 | 58 | 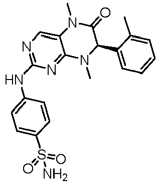 | 439.16 |
| 59 | 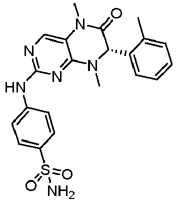 | 439.16 | | | |

### Example 1. Effects of various factors added to the culture medium for primary ovarian cancer cells on the proliferation of primary ovarian cancer cells

### (1) Preparation of culture mediums for primary ovarian cancer cells

First, a Basal Medium containing initial medium was prepared. The initial medium may be selected from the group consisting of DMEM/F12, DMEM, F12, or RPMI-1640 commonly used in the art. In this example, the formulation of the Basal Medium is: DMEM/F12 medium (purchased from Corning) + 100 µg/mL Primocin (purchased from InvivoGen, 0.2% (v/v), commercial product has a concentration of 50 mg/mL). Different types of additives (see Table 1) were added to the Basal Medium to prepare culture mediums for primary ovarian cancer cells with different components.

### (2) Isolation and treatment of primary ovarian cancer cells

### 1. Sample Selection

Tissue samples (intraoperative) of ovarian cancer solid tumor were obtained from patients by professional medical practitioner of professional medical institutions, and all patients have signed the informed consent. Intraoperative samples having the size of 0.25 cm³, endoscopic samples having the size of 0.025 cm³ were stored and transported using commercial tissue preservation solution (manufacturer: Miltenyi Biotec).

### 2. Material preparation

After subjecting to surface sterilization, 15 mL sterile centrifuge tubes, pipettors, 10 mL pipettes, sterile pipette tips, etc., were put in an ultra-clean workbench for ultraviolet irradiation of 30 minutes. The Basal Medium was taken out from a 4°C refrigerator 30 minutes in advance, and the tissue digestion solution was taken out from a -20°C refrigerator 30 minutes in advance.

Tissue digestion solution: 1640 medium (Corning, 10-040-CVR), collagenase II (2 mg/mL), collagenase IV (2 mg/mL), DNase (50 U/mL), hyaluronidase (0.75 mg/mL), calcium chloride (3.3 mM), BSA (10 mg/mL).

Collagenase II, collagenase IV, DNase, and hyaluronidase mentioned above were all purchased from Sigma Corporation; calcium chloride was purchased from Sangon Biotech (Shanghai) Co., Ltd.; BSA was purchased from Biofroxx Corporation.

### 3. Isolation of primary ovarian cancer cells

3.1. Tissue samples were transferred from the ultra-clean workbench to a culture dish, and the tissue with blood was removed. The tissue samples were rinsed twice with Basal Medium, and then were transferred to another culture dish and mechanically cut with a sterile scalpel into tissue blocks of 1×1×1 mm³ in size.

3.2. The cut intraoperative tissues were aspirated into a 15 mL centrifuge tube, to which 5 mL of Basal Medium was added, fully mixed, and then centrifuged at 1500 rpm for 4 minutes.

3.3. The supernatant was discarded, and the resultant was added with 1:3 mixture of Basal Medium and tissue digestion solution (the amount of tissue digestion solution added was about 10 mL tissue digestion solution for 1 g of tumor tissue). The samples were marked with names and numbers, sealed with sealing film, and then digested in a shaker (Zhichu Instrument ZQLY-180N) at 37°C, 300 rpm. The completion of digestion was determined via observation every 30 minutes, based on the existing of visible particles or not. The digestion time was 4 hours.

3.4. After the digestion was completed, undigested tissue mass was filtered out through a 100 µM filter mash. The tissue mass on the filter mash was rinsed into the centrifuge tube with Basal Medium to reduce cell loss. The resultant was centrifuged at 25°C, 1500 rpm for 4 minutes.

3.5. The supernatant was discarded and the resultant was observed to determine whether there were blood cells. If there were blood cells, 8 mL blood cell lysate (purchased from Sigma) was added in the resultant and fully mixed, lysed at 4°C for 20 minutes, with inverting and mixing once during the process. The resultant was centrifuged at 25°C, 1500 rpm for 4 minutes.

3.6. The supernatant was discarded and 2 mL Basal Medium was added to resuspend the cells for reserve.

### 4. Cell count and treatment

4.1. Microscopic observation: a small amount of resuspended cells were plated in a culture dish, and the density and morphology of cancer cells were observed under a microscope (CNOPTEC, BDS400);
4.2. Viable cell counting: 12 µL of the resuspended cell suspension was fully mixed with 12 µL of trypan blue staining solution (Sangon Biotech (Shanghai) Co., Ltd.), and then 20 µL of the mixture was added into a cell counting plate (Countstar, specifications: 50 pieces/box). The percentage of viable large cells (cell size > 10 µm) = number of viable cells / number of total cells × 100%, was calculated with a cell counter (Countstar, IC1000).

(3) Culture of primary ovarian cancer cellsThe primary ovarian cancer cells isolated from 2 cases of ovarian cancer tissues (numbered L40 and LQQ) according to the above step (2) were resuspended in the culture medium in Table 1, respectively and the cell number was counted. The Basal Medium was mixed with matrigel (Corning^{®}, 356231) in a 50:1 ratio on ice. 300 µL of the mixture was plated and incubated in a 5% CO₂ incubator at 37°C for 25-35 minutes, preferably 30 minutes. The supernatant was discarded. The culture medium with different components of Table 1 containing primary ovarian cancer cells was added to a 48-well plate with 4× 10⁴ cells per well and 500 µL/well. The Basal Medium without addition of any additive was used as a control. After 7-10 days of culture, the cells grew to 85%, and the culture mediums were discarded and the cells were rinsed once with 100 µL 0.05% trypsin (purchased from Gibco) per well. After aspirating the trypsin, 200 µL of 0.05% trypsin was added to each well. The plate was then placed in an incubator at 37 °C and 5% CO₂ for reaction of 10 minutes, and the cells were observed to be fully digested under a microscope (CNOPTEC, BDS400). Then, 300 µL of DMEM/F12 medium containing 10% serum (Excell Bio, FND500) was added to terminate the digestion, and 20 µL of resultant was added to a cell counting plate (Countstar, size: 50 pieces/box). The total number of cells was counted with a cell counter (Countstar, IC1000). The experimental results are shown in Table 1.

**Table 1: Additives in the culture mediums and their effects on promoting cell proliferation**

| Number | Types of medium additive | Supplier | Final concentration | Level of promoting proliferation |
|---|---|---|---|---|
| 1 | hepatocyte growth factor | Peprotech | 5 ng/mL | ○ |
| 2 | vitamin E | Sigma | 3 µg/mL | ○ |
| 3 | SB202190 | MCE | 7.5 µM | ○ |
| 4 | Compound 1 | Preparation Example | 20 µM | + |
| 5 | prostaglandin E2 | Tocris | 5 µM | - |
| 6 | noggin | R&D | 5 ng/mL | ○ |
| 7 | R-spondin1 | R&D | 125 ng/mL | ○ |
| 8 | hydrocortisone | Sigma | 5 ng/mL | ○ |
| 9 | B27 | Gibco | 1:100 (v/v) | - |
| 10 | N2 | Gibco | 1:100 (v/v) | + |
| 11 | SB431542 | MCE | 15 µM | ○ |
| 12 | epidermal growth factor | R&D | 10 ng/mL | - |
| 13 | insulin | Peprotech | 3 µg/mL | + |
| 14 | insulin-transferrin-selenium supplement | gibco | 1:50 (v/v) | + |
| 15 | A8301 | MCE | 27 nM | ○ |
| 16 | human fibroblast growth factor -10 | sino biological | 10 ng/mL | ○ |
| 17 | neuregulin -1 | MCE | 5 ng/mL | ○ |
| 18 | amphiregulin | MCE | 27 ng/mL | + |
| 19 | nicotinamide | Sigma | 1 mM | ○ |
| 20 | non-essential amino acid(s) | Corning | 50 µM | ○ |
| 21 | Y-27632 | MCE | 5 µM | + |
| 22 | basic fibroblast growth factor | novoprotein | 2.5 ng/mL | ○ |
| 23 | glutamine additive | thermo | 1: 100 (v/v) | ○ |
| 24 | insulin-like growth factor-1 | R&D | 50 ng/mL | + |
| 25 | forskolin | MCE | 10 µM | ○ |
| 26 | sodium pyruvate | Sigma | 0.5 mM | ○ |
| 27 | bovine pituitary extract | absin | 1:500 (v/v) | ○ |
| 28 | gastrin | MCE | 27 nM | - |
| 29 | CHIR99021 | MCE | 2.5 µM | ○ |
| 30 | fibroblast growth factors 7 | R&D | 5 ng/mL | ○ |
| 31 | cholera toxin | sigma | 0.1 µg/mL | + |

Wherein, "+" indicates that compared with the Basal Medium, the culture medium added with the additive(s) has the effect of promoting the proliferation of two cases of primary ovarian cancer cells isolated from ovarian cancer tissue; "-" indicates that the culture medium added with the additive(s) has the effect of promoting the proliferation of one case of primary ovarian cancer cells isolated from ovarian cancer tissue; "o" indicates that the culture medium added with the additive(s) has no significant effect on the proliferation of at least two cases of primary ovarian cancer cells isolated from ovarian cancer tissue.

According to the above results, factors such as Compound 1, prostaglandin E2, B27, N2, epidermal growth factor, insulin, insulin-transferrin-selenium supplement, amphiregulin, Y-27632, insulin-like growth factor-1, gastrin, cholera toxin were selected for further culture experiments.

Example 2 Effects of different combinations of added factors in the culture medium for primary ovarian cancer cells on the proliferation of primary ovarian cancer cells

The culture mediums for primary ovarian cancer cells containing different combinations of added factors were prepared according to the components in Table 2, to investigate the proliferation-promoting effects of different added factor combinations on primary ovarian cancer cells.

**Table 2 Preparation of culture mediums with different components (final concentrations are shown)**

| Culture medium | Composition |
|---|---|
| Basal Medium (BM) | DMEM/F12 + 100 µg/mL Primocin |
| No.1 | BM + 20 µM Compound 1 + 5 µM prostaglandin E2 + 1:100 (v/v) B27 + 1:100 (v/v) N2 + 10 ng/mL epidermal growth factor + 3 µg/mL insulin + 1:50 (v/v) insulin-transferrin-selenium supplement + 27 ng/mL amphiregulin + 5 µM Y-27632 + 50 ng/mL insulin-like growth factor-1 + 27 nM gastrin + 0.1 µg/mL cholera toxin |
| No.2 | No.1 - 20 µM Compound 1 |
| No.3 | No.1 - 5 µM prostaglandin E2 |
| No.4 | No.1 - 1:100 (v/v) B27 |
| No.5 | No.1 - 1:100 (v/v) N2 |
| No.6 | No.1 - 10 ng/mL epidermal growth factor |
| No.7 | No.1 - 3 µg/mL insulin |
| No.8 | No.1 - 1:50 (v/v) insulin-transferrin-selenium supplement |
| No.9 | No.1 - 27 ng/mL amphiregulin |
| No.10 | No.1 - 5 µM Y-27632 |
| No.11 | No.1 - 50 ng/mL insulin-like growth factor-1 |
| No.12 | No.1 - 27 nM gastrin |
| No.13 | No.1 - 0.1 µg/mL cholera toxin |

Primary ovarian cancer cells were obtained from ovarian cancer tissue (numbered L46, L55, L56) according to the process of step (2)-3 of Example 1. The obtained cell suspension was divided into 14 equal parts, which were then centrifuged at 1500 rpm for 4 minutes. After centrifugation, the cells were resuspended in 200 µL BM and culture mediums No. 1-13 respectively. The Basal Medium was mixed with matrigel (Corning^{®}, 356231) in a 50:1 ratio on ice. 300 µL of the mixture was plated in a 48-well plate and incubated in a 5% CO₂ incubator at 37°C for 25-35 minutes, preferably 30 minutes. The supernatant was discarded. The primary ovarian cancer cells were inoculated into the 48-well plate at a viable cell density of 4 × 10⁴ cells/well (40,000 cells per well). Each well in the 48-well plate was supplemented to a volume of 1 mL with the corresponding culture mediums, and the resultant was fully mixed. After subjecting to surface sterilization, the plate was placed in a 37°C, 5% CO₂ incubator (purchased from Thermo Fisher) for culture.

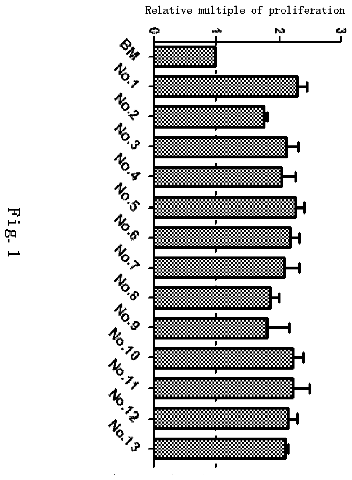

When the cells grew to more than 85% in the 48-well plate, the culture medium was discarded. The cells were rinsed once with 100 µL of 0.05% trypsin per well (purchased from Gibco). After aspirating the trypsin, 200 µL of 0.05% trypsin was added to each well. The plate was then placed in an incubator at 37 °C and 5% CO₂ for reaction of 10 minutes, and the cells were observed to be fully digested under a microscope (CNOPTEC, BDS400). Then, 300 µL of DMEM medium containing 10% fetal bovine serum was added to terminate the digestion, and 20 µL of the resultant was added to a cell counting plate (Countstar, specification: 50 pieces/box). The total number of cells was counted with a cell counter (Countstar, IC1000). The results obtained from primary ovarian cancer cells isolated from L46, L55, and L56 (intraoperative) are shown in Figure 1 .

According to the results in Figure 1, compared with the Basal Medium, the above culture mediums No. 1-13 may promote the proliferation of primary ovarian cancer cells to varying degrees. Therefore, when primary ovarian cancer cells are cultured with the culture medium containing additives such as Compound 1, prostaglandins E2, B27, N2, epidermal growth factor, insulin, insulin-transferrin-selenium supplement, amphiregulin, Y-27632, insulin-like growth factor-1, gastrin, cholera toxin, proliferation is better.

Example 3 Effects of different concentrations of added factors contained in the culture medium for primary ovarian cancer cells on the proliferation of primary ovarian cancer cells

Primary ovarian cancer cells were obtained from tissue samples (numbered A26083, L56, L62) according to the process of step (2)-3 of Example 1. The culture medium with a combination of the effective factors in Example 2 was used for culture. The Basal Medium was mixed with matrigel (Corning^{®}, 356231) in a 50:1 ratio on ice. 4 mL of the mixture was plated in a T12.5 culture flask and incubated in a 5% CO₂ incubator at 37 °C for 25-35 minutes, preferably 30 minutes. The supernatant was discarded.

The obtained primary ovarian cancer cells were seeded into a T12.5-well plate (300,000 cells per well) at a viable cell density of 2.4 × 10⁴ cells/cm². The plate was then placed in a 37°C, 5% CO₂ incubator (purchased from Thermo Fisher) after subjecting to surface sterilization. The cells were cultured and expanded using the culture medium with a combination of effective factors (containing Basal Medium (BM), 20 µM Compound 1, 5 µM prostaglandin E2, 1:100 (v/v) B27, 1:100 (v/v) N2 , 10 ng/mL epidermal growth factor, 3 µg/mL insulin, 1:50 (v/v) insulin-transferrin-selenium supplement, 27 ng/mL amphiregulin, 5 µM Y-27632, 50 ng/mL insulin-like growth factor-1, 27 nM gastrin, 0.1 µg/mL cholera toxin) determined in Example 2 until the cells grew to 85% or more, and 500 µL of 0.05% trypsin (purchased from Gibco) was added to rinse the cells for 1 minute. After aspirating the trypsin, 500 µL of 0.05% trypsin was then added to each well, and the plate was placed in a 37°C, 5% CO₂ incubator for reaction of 2-10 minutes. The digestion was terminated until the cells were completely digested. After centrifugation at 1500 rpm for 4 minutes, the supernatant was discarded. The cell pellet was resuspended in DMEM/F12, and then 20 µL of the suspension was added to a cell counting plate (manufacturer: Countstar, specification: 50 pieces/box), and the total number of cells was counted with a cell counter (Countstar, IC1000). The obtained cells were used in the following culture experiments.

The following 12 formulations of culture mediums were prepared for experiments.
Formulation 1: the above-mentioned culture medium for primary ovarian cancer cells without Compound 1;
Formulation 2: the above-mentioned culture medium for primary ovarian cancer cells without prostaglandin E2;
Formulation 3: the above-mentioned culture medium for primary ovarian cancer cells without B27;
Formulation 4: the above-mentioned culture medium for primary ovarian cancer cells without N2;
Formulation 5: the above-mentioned culture medium for primary ovarian cancer cells without epidermal growth factor;
Formulation 6: the above-mentioned culture medium for primary ovarian cancer cells without insulin;
Formulation 7: the above-mentioned culture medium for primary ovarian cancer cells without insulin-transferrin-selenium supplement;
Formulation 8: the above-mentioned culture medium for primary ovarian cancer cells without amphiregulin;
Formulation 9: the above-mentioned culture medium for primary ovarian cancer cells without Y-27632;
Formulation 10: the above-mentioned culture medium for primary ovarian cancer cells without insulin-like growth factor-1;
Formulation 11: the above-mentioned culture medium for primary ovarian cancer cells without gastrin;
Formulation 12: the above-mentioned culture medium for primary ovarian cancer cells without cholera toxin.

20 µl of cell resuspension containing 4 × 10⁴ cells was added to each well, which was then diluted with 1 mL culture medium of the above Formulations 1-12 respectively.

When using the culture medium of Formulation 1, the prepared Compound 1 was added to each well of the 48-well plate seeded with primary cells, with 1 mL per well, and the final concentrations of Compound 1 were 2.5 µM, 5 µM, 10 µM, 20 µM and 40 µM, respectively; and a well of Blank Control (BC) was set using the culture medium of Formulation 1.

When using the culture medium of Formulation 2, the prepared prostaglandin E2 was added to each well of the 48-well plate seeded with primary cells, with 1 mL per well, and the final concentrations of prostaglandin E2 were 2.5 µM, 5 µM, 10 µM, 20 µM and 40 µM, respectively; and a well of Blank Control (BC) was set using the culture medium of Formulation 2.

When using the culture medium of Formulation 3, the prepared B27 was added to each well of the 48-well plate seeded with primary cells, with 1 mL per well, and the final concentrations of B27 were 1:400 (V/V), 1:200 (V/V), 1:100 (V/V), 1:50 (V/V) and 1:25 (VlV), respectively; and a well of Blank Control (BC) was set using the culture medium of Formulation 3.

When using the culture medium of Formulation 4, the prepared N2 was added to each well of the 48-well plate seeded with primary cells, with 1 mL per well, and the final concentrations of N2 were 1:400 (V/V), 1:200 (V/V), 1:100 (V/V), 1:50 (V/V) and 1:25 (VlV), respectively; and a well of Blank Control (BC) was set using the culture medium of Formulation 4.

When using the culture medium of Formulation 5, the prepared epidermal growth factor was added to each well of the 48-well plate seeded with primary cells, with 1 mL per well, and the final concentrations of epidermal growth factor were 2.5 ng/mL, 5 ng/mL, 10 ng/mL, 20 ng/mL and 40 ng/mL, respectively; and a well of Blank Control (BC) was set using the culture medium of Formulation 5.

When using the culture medium of Formulation 6, the prepared insulin was added to each well of the 48-well plate seeded with primary cells, with 1 mL per well, and the final concentrations of insulin were 1 µg/mL, 3 µg/mL, 9 µg/mL, 27 µg/mL and 81 µg/mL, respectively; and a well of Blank Control (BC) was set using the culture medium of Formulation 6.

When using the culture medium of Formulation 7, the prepared insulin-transferrin-selenium supplement was added to each well of the 48-well plate seeded with primary cells, with 1 mL per well, and the final concentrations of insulin-transferrin-selenium supplement were 1:800 (V/V), 1:400 (V/V), 1:200 (V/V), 1:100 (V/V) and 1:50 (VlV), respectively; and a well of Blank Control (BC) was set using the culture medium of Formulation 7.

When using the culture medium of Formulation 8, the prepared amphiregulin was added to each well of the 48-well plate seeded with primary cells, with 1 mL per well, and the final concentrations of amphiregulin were 1 ng/mL, 3 ng/mL, 9 ng/mL, 27 ng/mL and 81 ng/mL, respectively; and a well of Blank Control (BC) was set using the culture medium of Formulation 8.

When using the culture medium of Formulation 9, the prepared Y-27632 was added to each well of the 48-well plate seeded with primary cells, with 1 mL per well, and the final concentrations of Y-27632 were 2.5 µM, 5 µM, 10 µM, 20 µM and 40 µM, respectively; and a well of Blank Control (BC) was set using the culture medium of Formulation 9.

When using the culture medium of Formulation 10, the prepared insulin-like growth factor-1 was added to each well of the 48-well plate seeded with primary cells, with 1 mL per well, and the final concentrations of insulin-like growth factor-1 were 12.5 ng/mL, 25 ng/mL, 50 ng/mL, 100 ng/mL and 200 ng/mL, respectively; and a well of Blank Control (BC) was set using the culture medium of Formulation 10.

When using the culture medium of Formulation 11, the prepared gastrin was added to each well of the 48-well plate seeded with primary cells, with 1 mL per well, and the final concentrations of gastrin were 1 nM, 3 nM, 9 nM, 27 nM and 81 nM, respectively; and a well of Blank Control (BC) was set using the culture medium of Formulation 11.

When using the culture medium of Formulation 12, the prepared cholera toxin was added to each well of the 48-well plate seeded with primary cells, with 1 mL per well, and the final concentrations of cholera toxin were 0.05 µg/mL, 0.1 µg/mL, 0.2 µg/mL, 0.4 µg/mL and 0.8 µg/mL, respectively; and a well of Blank Control (BC) was set using the culture medium of Formulation 12.

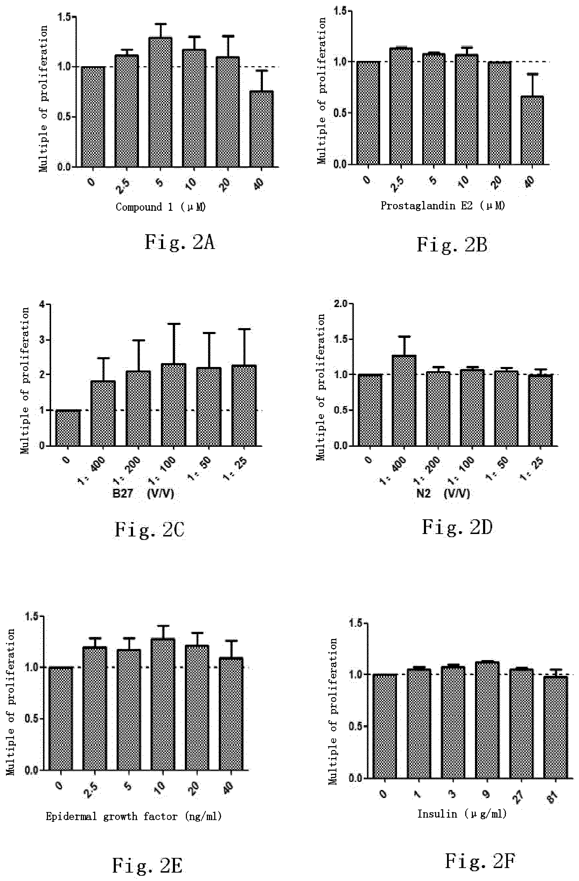

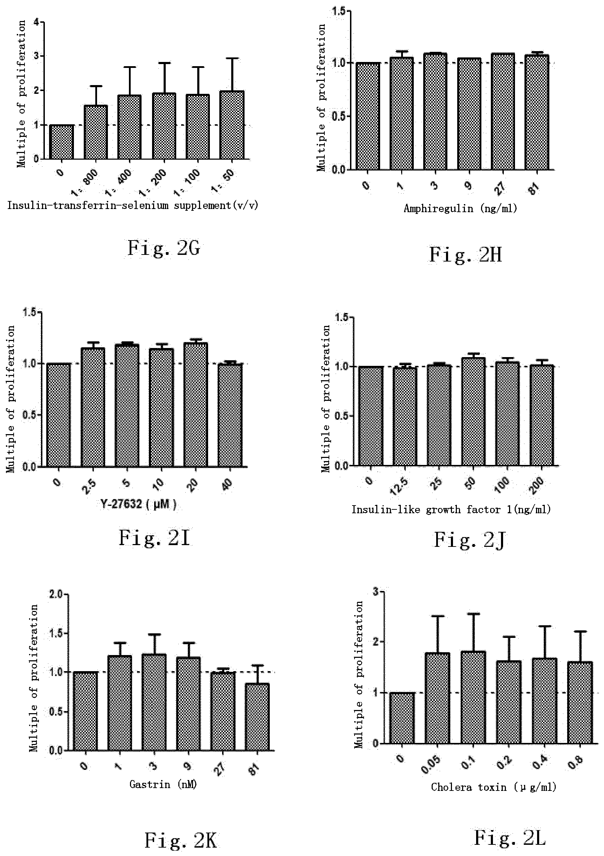

When the cells were expanded to about 85% of the 48 wells, the cells were digested and counted, and the proliferation multiple was calculated by referring to the number of cells in the well of Bland Control (BC), and the results were shown in Figure 2A-2L, respectively. In Figures 2A-2L, the ratio is a ratio of the number of cells of the first passage cultured by using each culture medium to the number of cells of the first passage cultured in the corresponding well of Blank Control. If the ratio is greater than 1, it indicates that the proliferation promoting effect of the prepared culture medium containing different concentrations of factors or small molecular compounds is preferable over that of the culture medium in the well of Blank Control; if the ratio is less than 1, it indicates that the proliferation promoting effect of the prepared culture medium containing different concentrations of factors or small molecular compounds is poorer than that of the culture medium in the well of Blank Control.

According to the results in Figures 2A-2L, Compound 1, prostaglandin E2, B27, N2, epidermal growth factor, insulin, insulin-transferrin-selenium supplement, amphiregulin, Y-27632, insulin-like growth factor-1, gastrin and cholera toxin have a significant proliferation-promoting effect on primary ovarian cancer cells. According to the results of this example, the amount of Compound 1 is preferably 2.5-20 µM, more preferably 2.5-10 µM, and the cell proliferation effect is most significant when the concentration is 5 µM; the amount of prostaglandin E2 is preferably 2.5-10 µM, and the cell proliferation effect is most significant when the concentration is 2.5 µM; the amount of B27 is preferably 1:25-1:400 (V/V), and more preferably 1:50-1:200 (VlV), and the cell proliferation effect is most significant when the concentration is 1:100 (V/V); the amount of N2 is preferably 1:50-1:400 (V/V), and the cell proliferation effect is most significant when the concentration is 1:400 (V/V); the amount of epidermal growth factor is preferably 2.5-40 ng/mL, more preferably 5-20 ng/mL, and the cell proliferation effect is most significant when the concentration is 10 ng/mL; the amount of insulin is preferably 1-27 µg/mL, and more preferably 3-9 µg/mL, and the cell proliferation effect is most significant when the concentration is 9 µg/mL; the amount of insulin-transferrin-selenium supplement is preferably 1:50-1:800 (V/V), more preferably 1:400-1:100 (V/V), and the cell proliferation effect is most significant when the concentration is 1:200 (V/V); the amount of amphiregulin is preferably 1-81 ng/mL, more preferably 1-27 ng/mL, and the cell proliferation effect is most significant when the concentration is 3 ng/mL; the amount of Y-27632 is preferably 2.5-20 µM, and the cell proliferation effect is most significant when the concentration is 5 µM; the amount of insulin-like growth factor-1 is preferably 25-100 ng/mL, and the cell proliferation effect is most significant when the concentration is 50 ng/mL; the amount of gastrin is preferably 1-9 nM, and the cell proliferation effect is most significant when the concentration is 3 nM; the amount of cholera toxin is preferably 0.05-0.8 µg/mL, more preferably 0.05-0.2 µg/mL, and the cell proliferation effect is most significant when the concentration is 0.1 µg/mL.

Using the most preferred concentrations of each added factor in the above culture medium, the culture medium for primary ovarian cancer cells of the invention used in the following examples contains: Basal Medium (BM), 5 µM Compound 1, 2.5 µM prostaglandin E2, 1:100 (V/V) B27, 1:400 (V/V) N2, 10 ng/mL epidermal growth factor, 9 µg/mL insulin, 1:200 (V/V) insulin-transferrin-selenium supplement, 3 ng/mL amphiregulin, 5 µM Y-27632, 50 ng/mL insulin-like growth factor-1, 3 nM gastrin, 0.1 µg/mL cholera toxin (hereinafter referred to as culture medium "OC-2").

### Example 4 Culture of primary ovarian cancer cells

Primary ovarian cancer cells were obtained from 6 cases of intraoperative tissue samples (numbered A26083, L65, L66, L72, L74, L84) according to the process of step (2)-3 of Example 1, and cultured using culture medium OC-2. The Basal Medium was mixed with matrigel (Corning^{®}, 356231) in a 50:1 ratio on ice. 2 mL of the mixture was plated in a 6-well plate and incubated at 37 °C in a 5% CO₂ incubator for 25-35 minutes, preferably 30 minutes, and the supernatant was discarded. The obtained primary ovarian cancer cells were seeded into a 6-well plate (250,000 cells per well) at a viable cell density of 2 × 10⁴ cells/cm², and 4 mL of culture medium OC-2 was added. After surface sterilization, the plate was placed in a 37°C, 5% CO₂ incubator (purchased from Thermo Fisher) for culture.

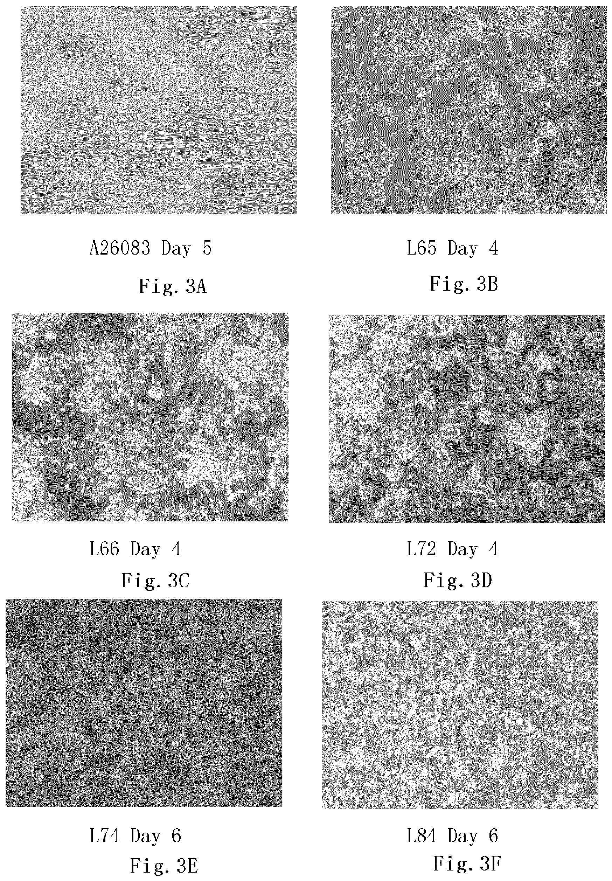

The cultured primary ovarian cancer cells were observed under the microscope (EVOS M500, Invitrogen). Figures 3A to 3F are photos of primary ovarian cancer cells obtained from samples A26083, L65, L66, L72, L74, and L84 after being cultured for 4-6 days respectively, taken under a 10x objective lens. The cells were closely arranged and slightly irregular in shape under the microscope.

### Example 5 Histochemical identification of culture of primary ovarian cancer cells

A cancer tissue of approximately 0.25 cm³ was removed from the intraoperative tissue (sample number L74) of a patient with ovarian cancer and fixed by immersing in 1 mL of 4% paraformaldehyde. The method of Example 4 was used to continue culturing sample L74 to the fourth passage using the culture medium OC-2 for primary ovarian cancer cells of the invention. Primary ovarian cancer cells fixed in 4% paraformaldehyde were embedded in paraffin and cut into 4 µm thick tissue sections using a microtome. Routine immunohistochemical detection was then performed (for specific procedures, see Li et al., Nature Communication, (2018) 9: 2983). The primary antibodies used were ER, PR, P53, NapsinA, Pax-8, WT-1, and Ki-67 (all purchased from CST).

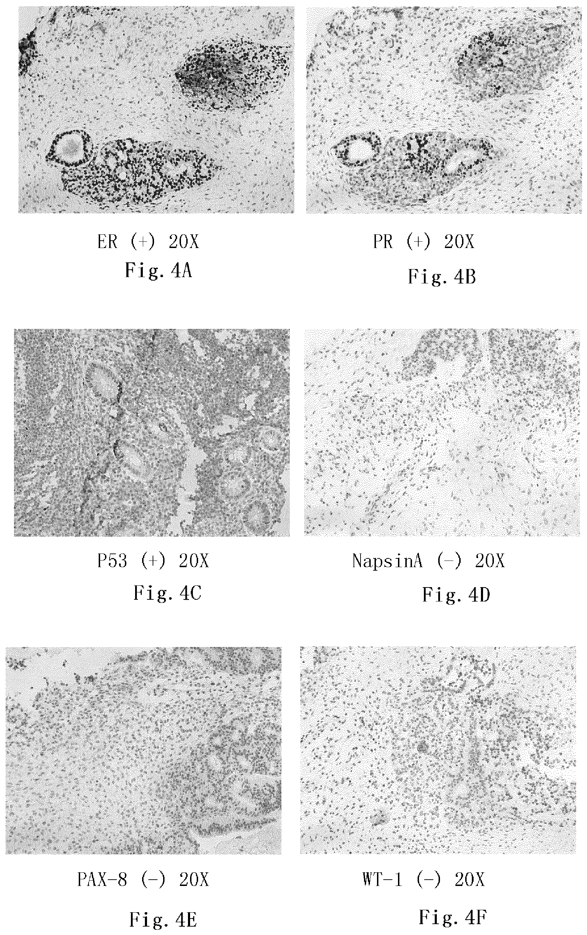

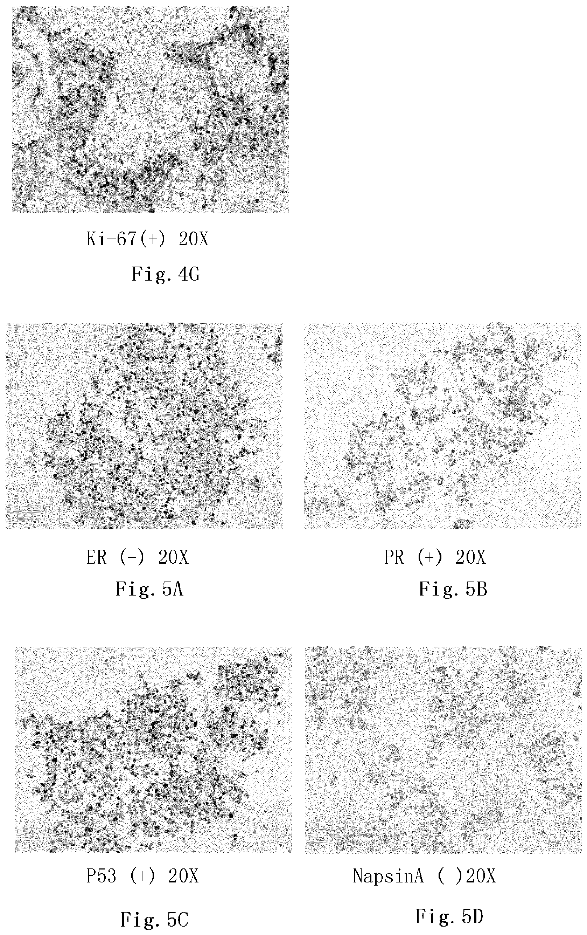

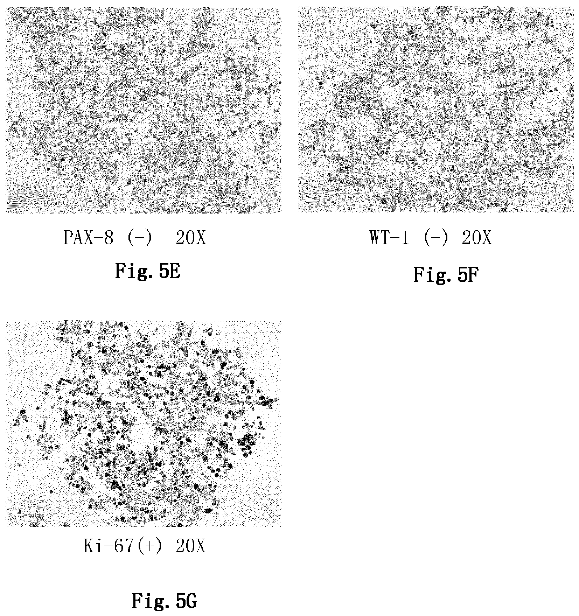

Figures 4A-4G and 5A-5G are comparative pictures of immunohistochemical results of original tissue cells and the primary ovarian cancer cells obtained by culturing the original tissue cells in the culture medium OC-2 for primary ovarian cancer cells of the invention, respectively. Figure 4A and Figure 5A are pictures of ER antibody labeling in ovarian cancer tissue and cultured primary ovarian cancer cells respectively. Figure 4B and Figure 5B are pictures of PR antibody labeling in ovarian cancer tissue and cultured primary ovarian cancer cells respectively. Figure 4C and Figure 5C are pictures of P53 antibody labeling in ovarian cancer tissue and cultured primary ovarian cancer cells respectively. Figure 4D and Figure 5D are pictures of NapsinA antibody labeling in ovarian cancer tissue and cultured primary ovarian cancer cells respectively. Figure 4E and Figure 5E are pictures of Pax-8 antibody labeling in ovarian cancer tissue and cultured primary ovarian cancer cells. Figure 4F and Figure 5F are pictures of WT-1 antibody labeling in ovarian cancer tissue and cultured primary ovarian cancer cells respectively. Figures 4G and 5G are pictures of Ki-67 antibody labeling in ovarian cancer tissue and cultured primary ovarian cancer cells. Thus, it can be confirmed that when the primary ovarian cancer cells cultured using the technology of the invention are cultured to the fourth passage, the expression of biomarkers related to ovarian cancer on the primary ovarian cancer cells is consistent with that of the original tissue slice from which the primary ovarian cancer cells are derived. It indicates that the primary ovarian cancer cells cultured by the technology of the invention maintain the original pathological characteristics of the cancer tissue of ovarian cancer patients.

### Example 6 Comparison with the culture effect of existing culture mediums, statistics on the culture cycle and cell number of primary ovarian cancer cells, and calculation of Population Doubling (PD) value

The prior art culture medium (Xuefeng Liu et al., Nat. Protoc., 12(2): 439-451, 2017)), which has the formulation of: DMEM/F12 medium + 250 ng/mL amphotericin B (purchased from Selleck) + 10 µg/mL gentamicin (purchased from MCE) + 0.1 nM cholera toxin + 0.125 ng/mL EGF + 25 ng/mL hydrocortisone + 10 µM Y27632 + 10% FBS.

### Commercial culture medium: Defined K-SFM, Keratinocyte Serum Medium (purchased from gibco, 10744-019)

Primary ovarian cancer cells were obtained from three cases of ovarian cancer tissue samples (numbered L65, L66, and A22083) according to the process of step (2)-3 of Example 1. The obtained primary ovarian cancer cells were cultured using the prior art culture medium, commercial culture medium and the culture medium OC-2 in Example 3 respectively, and the cells were seeded in a 6-well plate at a viable cell density of 3×10⁴ cells/cm². After the cells were expanded to 95%, they were digested and counted, and the days in culture until digestion was recorded as a culture cycle. Under this experimental condition, the expanded cells were expanded in different passages. The cells of each passage were digested and counted and the corresponding culture cycle was recorded. PD value was calculated according to the formula, Population Doubling (PD) = 3.32*log10 (total number of cells after digestion / initial number of inoculated cells). For the formula, see Chapman et al., Stem Cell Research & Therapy 2014, 5: 60.

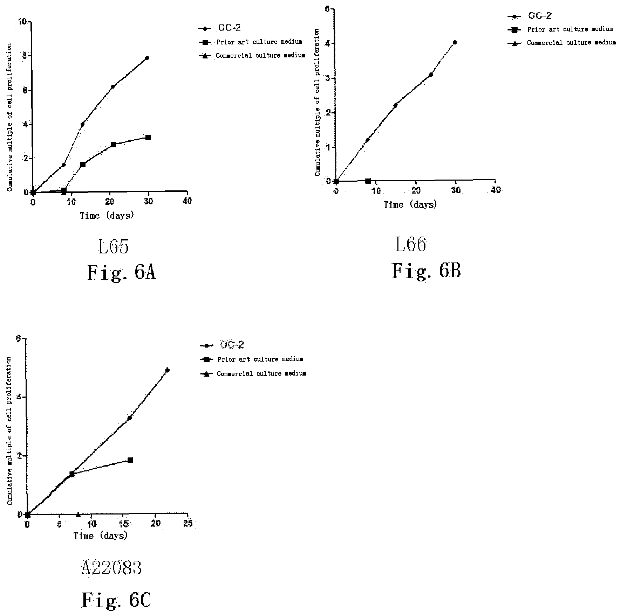

Figures 6A-6C show the growth curves of three cases of primary cells cultured using the prior art culture medium, the commercial culture medium and culture medium OC-2 for primary ovarian cancer cells of the invention drawn by Graphpad Prism software. The abscissa represents the days of cell culture, and the ordinate represents the cumulative multiple of cell proliferation, i.e., the multiple of cell expansion in the culture cycle. The larger the value, the more times the cells are expanded within a certain cycle, that is, the more cells are expanded. The slope represents the rate of cell expansion. It can be confirmed from Figures 6A-6C that when the primary ovarian cancer cells cultured in the culture medium OC-2 of the invention are continuously cultured and expanded for at least 30 days, the cell expansion rate remains basically unchanged and the cells still have the ability to continue to expand. The results in Figures 6A-6C show that the expansion rate of primary ovarian cancer cells cultured using the prior art culture medium and the commercial medium is significantly lower than that in OC-2 medium. In summary, compared with the prior art culture medium and the commercial medium, the proliferation efficiency of ovarian cancer cells cultured *in vitro* using the culture medium for primary ovarian cancer cells of the invention is significantly better.

### Example 7 Use of primary ovarian cancer cells expanded using the culture medium of the invention for drug screening and efficacy evaluation

### 1. Culture and plating of cells

Primary ovarian cancer cells (numbered A22083) were isolated according to the process of step (2)-3 of Example 1, used as a generation, and were cultured with the culture medium OC-2 for primary ovarian cancer cells of the invention until the cells were expanded to 85% and passaged. The cells were passaged and counted according to step (2)-4 in Example 1. The cells were placed in a loading slot (purchased from Corning) at a viable cell density of 1 × 10⁵ cells/mL. After being thoroughly mixed, they were placed in a 384-well opaque white cell culture plate (purchased from Corning), with a volume of 50 µL per well and a cell number of 5,000 cells/well. The plate was sealed by adding the culture medium for primary ovarian cancer cells of the invention from the edge of the plate, and the sample names, dosing times and testing times of CellTiter-Glo (Promega) were marked on the plate. The surface of the plate was disinfected with 75% alcohol (LIRCON), and the plate was cultured in a 37°C, 5% CO₂ incubator, and dosed 24 hours later. Cells of the 1st, 2nd, 3rd, 4th and 5th passages of culture were respectively obtained for drug screening, and the drug sensitivity of the primary ovarian cancer cells cultured using the culture medium of the invention for continuous passage was tested.

### 2. Preparation of candidate drugs

Six drugs (Cytarabine, Doxorubicin, Bortezomib, Panobinostat, Azacitidine, and Homoharringtonine; all purchased from MCE) in 6 concentration gradients were prepared according to the following table, which were added to a 384-well plate (Thermo Fisher) in a volume of 30 µL per well and stored for use.

**Table 3 Settings of drug concentration**

| No. | Drug names | Concentration gradient (µM) | | | | | |
|---|---|---|---|---|---|---|---|
| 1 | Cytarabine | 7.26 | 3.63 | 1.815 | 0.9075 | 0.45375 | 0.226875 |
| 2 | Doxorubicin | 0.062 | 0.031 | 0.0155 | 0.00775 | 0.003875 | 0.0019375 |
| 3 | Bortezomib | 0.52 | 0.26 | 0.13 | 0.065 | 0.0325 | 0.01625 |
| 4 | Panobinostat | 0.094 | 0.047 | 0.0235 | 0.01175 | 0.005875 | 0.0029375 |
| 5 | Azacitidine | 34 | 17 | 8.5 | 4.25 | 2.125 | 1.0625 |
| 6 | Homoharringtonine | 0.56 | 0.28 | 0.14 | 0.07 | 0.035 | 0.0175 |

### 3. High-throughput dosing

The plate with prepared drugs was taken out and kept at room temperature. The plate was centrifuged at room temperature for 1 minute at 1,000 rpm in a centrifuge (Beckman), and then taken out from the centrifuge. A high-throughput automated workstation (JANUS, Perkin Elmer) was used for high-throughput dosing. To each well of the 384-well plate with cultured primary ovarian cancer cells was added 0.1 µL of the candidate drugs of corresponding concentrations. After dosing, the surface of 384-well plate was disinfected and moved to a incubator. The cell viability was measured after 72 hours.

### 4. Measurement of cell viability

CellTiter-Glo luminescent reagent (Promega) was taken out from a 4°C refrigerator, and 10 mL of the reagent was added into the loading slot. The 384-well plate to be tested was taken out from the incubator, and 10 µL CellTiter-Glo luminescent reagent was added into each well. After being standed for 10 minutes, the test was conducted by using a multifunctional microplate reader (Envision, Perkin Elmer).

### 5. Data processing

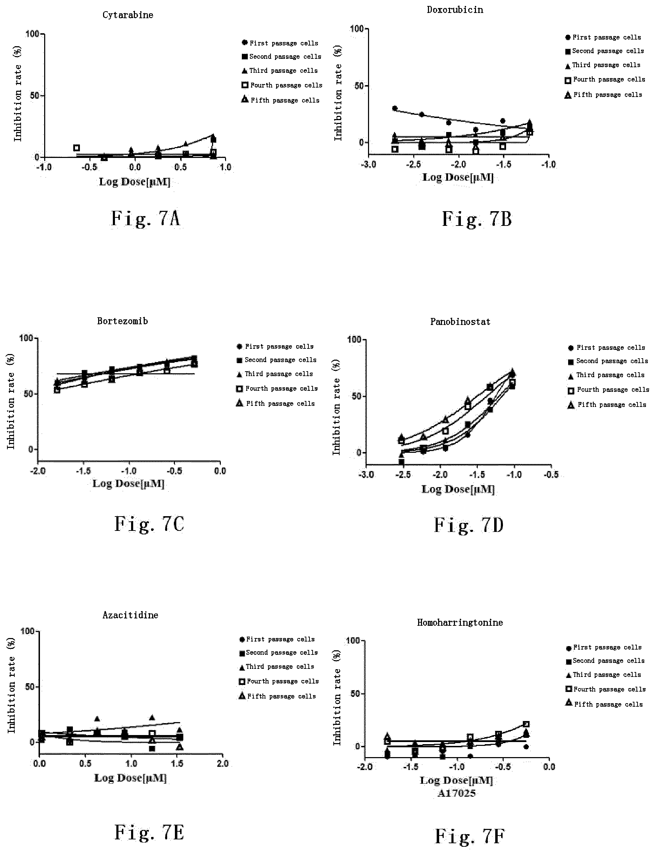

According to the formula, cell inhibition rate (%) = 100% - chemiluminescence value of the drug-dosed well / chemiluminescence value of the control well * 100%, the cell inhibition rate of cells treated with different drugs was calculated, and the half-inhibition rate (IC₅₀) of drugs on cells was calculated by the graphpad prism software. The results are shown in Figures 7A-7F.

It can be confirmed from Figures 7A-7F that when the primary ovarian cancer cells cultured from the culture medium OC-2 for primary ovarian cancer cells of the invention were used for drug screening, the inhibitory effects of the same drug on the cultured cells of different passages are substantially consistent (the inhibition curve are substantially consistent). The cells from the same patient have different sensitivities to different drugs at the maximum blood concentration in the human body. According to the results, the effectiveness of the drug in clinical use in patients with ovarian cancer can be judged. At the same time, the results indicate that the sensitivity of tumor cells of different passages obtained according to the culture method of the invention to the drugs is stable.

### Industrial Applicability

The invention provides a primary cell culture medium and a culture method for culturing primary ovarian cancer cells *in vitro,* and the cultured cells can be used for efficacy evaluation and screening of drugs. Therefore, the invention is suitable for industrial applications.

Although the invention has been described in detail herein, the invention is not limited thereto, and those skilled in the art can make modifications according to the principle of the invention. Therefore, any modification made in accordance with the principle of the invention shall be understood as falling within the protection scope of the invention.

## Claims

1. A culture medium for primary ovarian cancer cells, **characterized in that**, comprising:
an MST1/2 kinase inhibitor, at least one Rho kinase inhibitor selected from the group consisting of Y27632, fasudil, and H-1152; insulin-transferrin-selenium supplement; insulin; prostaglandin E2; epidermal growth factor; gastrin; insulin-like growth factor-1; cholera toxin; amphiregulin; N2; and B27,
wherein the MST1/2 kinase inhibitor comprises a compound of Formula (I) or a pharmaceutically acceptable salt, or a solvate thereof, 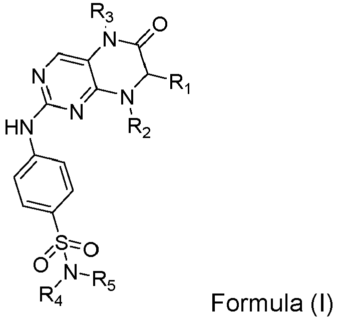 wherein,
R₁ is selected from C1-C6 alkyl, C3-C6 cycloalkyl, C4-C8 cycloalkylalkyl, C2-C6 spirocycloalkyl, and aryl optionally substituted with 1-2 independent R₆, aryl C1-C6 alkyl optionally substituted with 1-2 independent R₆ and heteroaryl optionally substituted with 1-2 independent R₆;
R₂ and R₃ are each independently selected from C1-C6 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl;
R₆ is selected from halogen, C1-C6 alkyl, C1-C6 alkoxy, and C1-C6 haloalkyl.

2. The culture medium of claim 1, wherein,
R₁ is selected from C1-C6 alkyl, C3-C6 cycloalkyl, C4-C8 cycloalkylalkyl, C2-C6 spirocycloalkyl, and phenyl optionally substituted with 1-2 independent R₆, naphthyl optionally substituted with 1-2 independent R₆, phenylmethyl optionally substituted with 1-2 independent R₆ and thienyl optionally substituted with 1-2 independent R₆;
R₂ and R₃ are each independently selected from C1-C3 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, piperidyl C1-C6 alkyl, and tetrahydropyranyl C1-C6 alkyl;
R₆ is selected from halogen, C1-C6 alkyl, C1-C6 alkoxy, and C1-C6 haloalkyl.

3. The culture medium of claim 1, wherein the MST1/2 kinase inhibitor comprises a compound of Formula (Ia) or a pharmaceutically acceptable salt, or a solvate thereof, 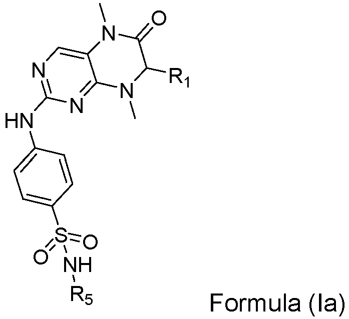 wherein,
R₁ is selected from C1-C6 alkyl, phenyl optionally substituted with 1-2 independent R₆, thienyl optionally substituted with 1-2 independent R₆, and phenylmethyl optionally substituted with 1-2 independent R₆;
R₅ is selected from hydrogen, C1-C6 alkyl, and C3-C6 cycloalkyl;
R₆ is independently selected from halogen, C1-C6 alkyl, and C1-C6 haloalkyl.

4. The culture medium of claim 3, wherein
R₁ is phenyl optionally substituted with 1-2 independent R₆;
R₅ is hydrogen;
R₆ is fluoro, methyl or trifluoromethyl.

5. The culture medium of claim 1, wherein the MST1/2 kinase inhibitor is at least one selected from the following compounds or a pharmaceutically acceptable salt thereof:
| | | | |
|---|---|---|---|
| 1 | 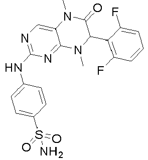 | 2 | 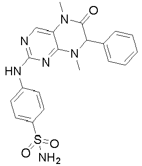 |
| 3 | 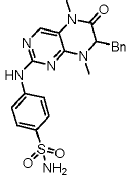 | 4 | 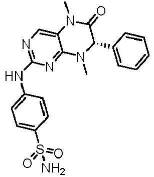 |
| 5 | 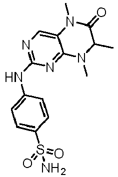 | 6 | 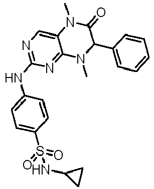 |
| 7 | 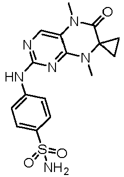 | 8 | 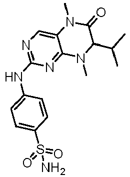 |
| 9 | 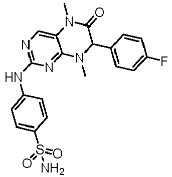 | 10 | 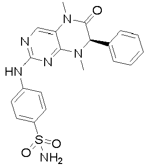 |
| 11 | 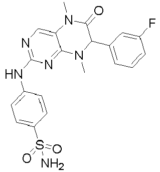 | 12 | 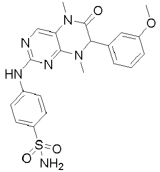 |
| 13 | 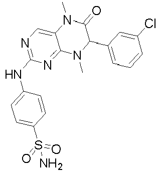 | 14 | 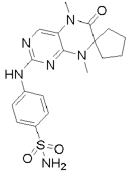 |
| 15 | 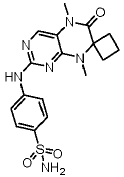 | 16 | 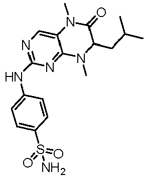 |
| 17 | 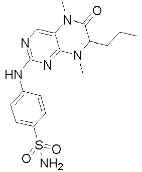 | 18 | 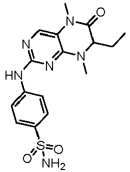 |
| 19 | 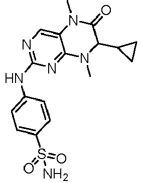 | 20 | 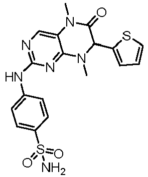 |
| 21 | 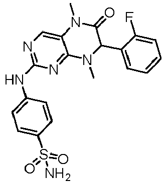 | 22 | 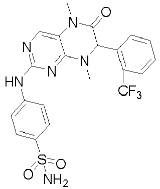 |
| 23 | 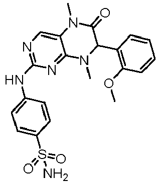 | 24 | 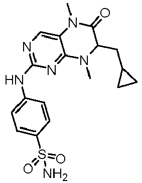 |
| 25 | 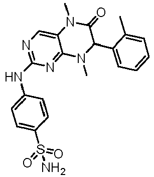 | 26 | 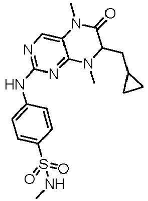 |
| 27 | 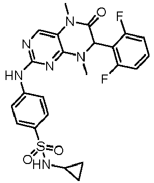 | 28 | 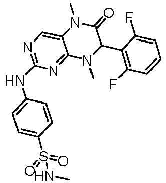 |
| 29 | 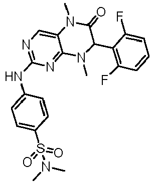 | 30 | 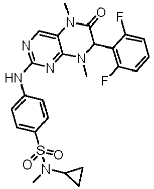 |
| 31 | 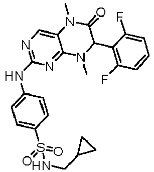 | 32 | 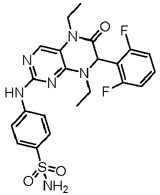 |
| 33 | 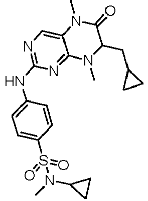 | 34 | 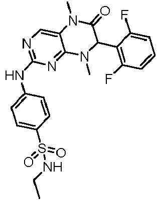 |
| 35 | 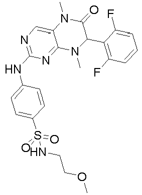 | 36 | 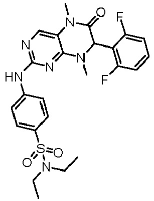 |
| 37 | 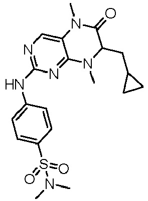 | 38 | 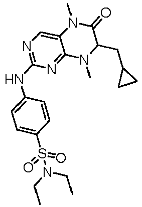 |
| 39 | 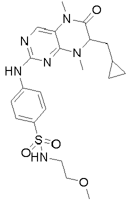 | 40 | 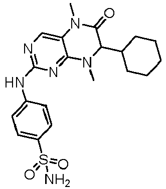 |
| 41 | 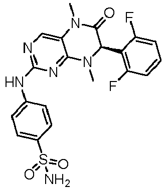 | 42 | 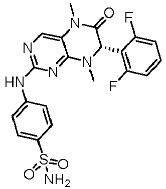 |
| 43 | 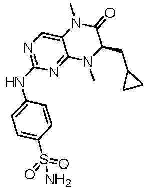 | 44 | 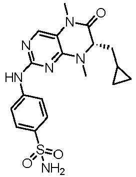 |
| 45 | 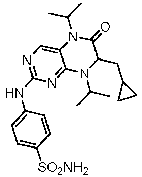 | 46 | 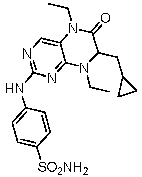 |
| 47 | 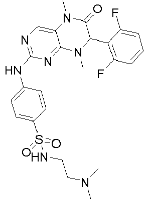 | 48 | 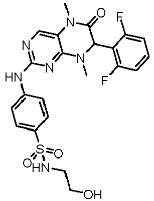 |
| 49 | 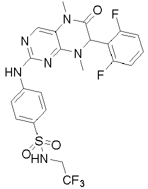 | 50 | 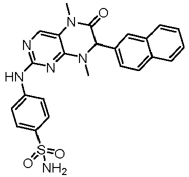 |
| 51 | 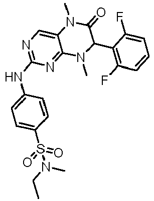 | 52 | 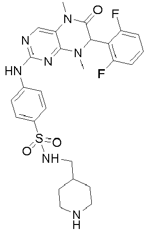 |
| 53 | 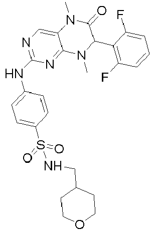 | 54 | 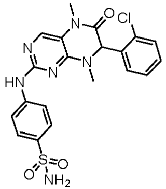 |
| 55 | 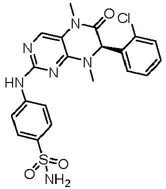 | 56 | 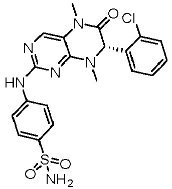 |
| 57 | 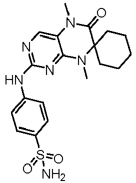 | 58 | 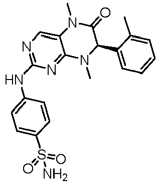 |
| 59 | 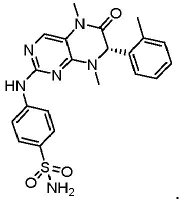 | | |

6. The culture medium of any one of claims 1-5, **characterized in that**, the amount of the components in the culture medium satisfies any one or more or all of the following conditions:
(1) the amount of the MST1/2 kinase inhibitor in the culture medium is 2.5-20 µM;
(2) the amount of the Rho kinase inhibitor in the culture medium is 2.5-20 µM;
(3) the volume ratio of the insulin-transferrin-selenium supplement relative to the culture medium is 1:800-1:50;
(4) the amount of the insulin in the culture medium is 1-27 µg/mL;
(5) the amount of the prostaglandin E2 in the culture medium is 2.5-10 µM;
(6) the amount of the epidermal growth factor in the culture medium is 2.5-40 ng/mL;
(7) the amount of the gastrin in the culture medium is 1-9 nM;
(8) the amount of the insulin-like growth factor-1 in the culture medium is 25-100 ng/mL;
(9) the amount of the cholera toxin in the culture medium is 0.05-0.8 µg/mL;
(10) the amount of the amphiregulin in the culture medium is 1-81 ng/mL;
(11) the volume ratio of the B27 additive relative to the culture medium is preferably 1:25-1:400;
(12) the volume ratio of the N2 additive relative to the culture medium is preferably 1:50-1:400.

7. The culture medium of any one of claims 1-6, **characterized in that**, further comprising:
an initial medium selected from the group consisting of DMEM/F12, DMEM, F12 or RPMI-1640; and one or more antibiotics selected from the group consisting of streptomycin/penicillin, amphotericin B and Primocin.

8. A method for culturing primary ovarian cancer cells, **characterized in that**, comprising the following steps:
(1) preparing the culture medium for primary ovarian cancer cells of any one of claims 1-7;
(2) obtaining primary ovarian cancer cells from ovarian cancer tissue samples;
(3) adding the culture medium for primary ovarian cancer cells obtained in step (1) to the primary ovarian cancer cells obtained in step (2) for culture.

9. A method for evaluating or screening a drug for treating ovarian cancer, **characterized in that**, comprising the following steps:
(1) culturing primary ovarian cancer cells using the method for culturing primary ovarian cancer cells of claim 8;
(2) selecting the drug to be tested and diluting the drug into desired concentration gradients;
(3) adding the drug which has been diluted into various concentration gradients to the primary ovarian cancer cells cultured in step(1);
(4) detecting the cell viability.
